(19) Europäisches Patentamt / European Patent Office / Office européen des brevets

(11) **EP 3 401 873 A1**

(12) **EUROPEAN PATENT APPLICATION**
published in accordance with Art. 153(4) EPC

(43) Date of publication:
**14.11.2018 Bulletin 2018/46**

(51) Int Cl.:
*G06Q 50/22* (2018.01)      *A61B 5/103* (2006.01)

(21) Application number: **15908228.8**

(22) Date of filing: **09.12.2015**

(86) International application number:
**PCT/HU2015/000078**

(87) International publication number:
**WO 2017/081497 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.11.2015 HU 1500528**

(71) Applicant: **AXS Motionsystem Kft.**
**1045 Budapest (HU)**

(72) Inventors:
• SZÉKELY, Lajos
  **H-2151 Fót (HU)**
• RABOCZKI, Zoltán
  **H-1038 Budapest (HU)**
• ANDOR, László
  **H-1031 Budapest (HU)**

(74) Representative: **Kaszás, Eszter**
**Maros utca 28, 1. em. 4**
**1122 Budapest (HU)**

(54) **DEVICE FOR DIGITIZING AND EVALUATING MOVEMENT**

(57)      A motion digitizing and evaluating device, capable of preparing recordings or motion files more accurate - from several aspects - than those known so far, even without a permanent external reference signal.

In addition to the inertial measurement units fixed to the separately moving body parts or spare parts of the examined person, living creature or objects (2, 3, 4, 5, 6, 7, 8, 9, 10, 11, 12, 13, 14, 15, 16, 17, 18), basic parts of the device are also the pressure-sensitive sole units (19, 20) and the pressure sensor gloves (21, 22), thanks to which the location-changing movements can be truly tracked by the virtual bone system mapped by the software.

With more accurate motion detection, the external magnetic oriantation - which has so far been inevitable for such purposes in the previous systems - or the application of multi-axial magnetometers can be easily eliminated. A part of the patent is a strap vest for the more precise fixing of the strain-related sensors on the back.

15.   Figure

**Description**

The subject matter of the patent is a motion digitizing and evaluating device, capable of preparing recordings or motion files more accurate - from several aspects - than those known so far, even without a permanent external reference signal. A processor software is closely linked to the device as a hardware. An important element of the device is the sensor net operating based on the inertia system and closely fitted to the body, on the basis of the signals of which the processor software - capable of immediate evaluation - maps the spatial movement of the examined body. The device can be used for ergonomic analysis, motion animation, in the entertainment industry, healthcare, analysis of athletes or other purposes.

During the 3D motion digitization, the movement of the person observed (or even other creatures or objects with a greater level of freedom) is recorded, then a digital model is created that traps the movement of the subject according to the required resolution. On the basis of the recording, lifelike animated movies, ergonomic recordings or motion pictures suitable for analysis can be prepared and organized into searchable databases.

In a simpler case, only the movement of the subject is recorded. Records of the currently known MOCAP systems can be made with conventional or infrared cameras or acoustic recorders placed at multiple locations in the space. An example of this is the description of the patent titled 'EP2787456 (A2) - Creating ergonomic manikin postures and controlling computer-aided design environments using natural user interfaces'. It is also known to use passive or active markers placed on important points of the subject's body that are followed by multiple cameras or an acoustic or magnetic recorder corresponding to the markers, and then the computer calculates the position and displacement of the subject's skeleton by triangulation, from the position of the markers. In one of their simplest form, markers may also be inertial encoders fixed to body parts forming an independent movement unit. In this case, no external monitoring device is required and a vector frame can be compiled based on the sensors' signals, which is clearly defined on the basis of the angular position of each limb. An example for this design is the sensor system of the patent titled US2007250286 (A1). - Motion Monitoring and Analysis System, which, although having a large number of sensors, including also some sole pressure sensors and strain gauging stamps on the sole, thereby mapping motion with satisfactory fidelity, but the disadvantage of it is that at least one of the trunk sensors is not on the back, which ameliorates the accuracy of motion sensing. The device is recommended to monitor the physical integrity of physical workers (especially the spine), and the calculation is based on the value of the lifted weight measurable from the posture sensation and the sole pressure. The signals of the sole sensors do not take part in the calculation of motions, and also because of the area of use, the mapped model does not perform any location-changing movement, nor is it necessary to monitor it. The patent titled US2013217352 (A1) - SYSTEM AND METHOD TO PREDICT AND AVOID MUSCULOSKELETAL INJURIES sees the sole pressure for a similar purpose in addition to the body position, and, in addition, this is performed at three points, but it takes place strictly for the measurement of the spinal load here, as well, and the location changing movements are not calculated. From the patent titled WO2012161407 (A1) - METHOD OF MOTION TRACKING, again a system consisting of intertial encoders fixed to the body can be known, but here again, at least one of the trunk sensors is placed on the abdominal side, deteriorating sensing. In case of use for ergonomic, healthcare or sports purposes, it is often necessary not only to track the limbs' position but also that of the external forces that awake on them. In case of industrial application, e.g. sensing or monitoring the pressures arising out of the hold awakening on the surface of the hand or for other reasons, and it is often necessary to follow the location-changing movement of the subject, as well. The latter task is often performed by the traditional motion digitizing systems by using an extra sensor bound to an external reference, e.g. a magnetometer, which, however, is inaccurate and unusable in the vicinity of larger metal objects (machines). Another disadvantage of the known motion digitizing and evaluating systems is that they track the walking of subjects inaccurately, not enough smoothly and imprecisely by moving the model legs consisting only of 3 vectors, and, as a result, the spatial position of the subject not performing a stationary work becomes uncertain in time and the mapped model differs from reality.

Our goal is to overcome the motion digitizing and evaluating systems based on the known intertial encoders, primarily by more accurate mapping, including also more accurate tracking of location-changing movements. Our further goal is to track the static and dynamic states more accurately, to identify them and to achieve as much autonomous operation as possible. In addition to this, our goal is to sense the external forces awakening at the primary contact surfaces of the body (sole, hand) simultaneously with the body position, as this mainly provides important information in industrial and ergonomic applications.

Our proposed solution is based on the recognition that the signals of the sole pressure measuring devices, applied for other purposes (mainly for load measurement) can be perfexctly used also for analyzing movements. With their help, it is possible to find out more accurately, when the legs are in a stabile position on the ground, and also the distance made can be measured more accurately than before. By using pressure sensors, it can be easily detected whether a given person is standing on two legs, it can be specified whether he or she is in a static or dynamic state, and on the basis of the different pressure between the heel and the toes, the moment of leg changes can be determined more accurately. The recommended device can distinguish exactly when the first third of the sole and the heel is on the ground, thus more detailed information can be received on the process of walking than those provided by the previous systems.

When the walking process starts, first the heel of the stepping leg rises from the ground and the first part of the sole remains motionless, until it rises from the ground. By integrating the pressure sensors into a system, one can determine the angle difference between the first and second third of the sole, making the animation of the human model more precise and smooth. Thanks to the pressure sensors, the differentiation of dynamic and static states and the separation of the body of the measured subject from a vertical surface can be feedbacked, too. Because of the more precise motion sensing, it is easy to omit the external magnetic orientation that has been essential for this purpose in the systems used so far. This sensing method has been operating difficultly in industrial environments either ways, due to the distoring effect of larger metal objects. A further recognition is that instead of the three sensors that have been so far applied by the previous systems, the movement of the spine is recorded by four sensors, and, as a result of the special strap vest suggested by us, all of the sensors lie directly on the spine, none of them being placed on the chest, so that the position of the spine can be tracked more accurately.

The subject matter of the proposed patent is therefore a motion digitizing and evaluating device, consisting of intertial measurement units fixed on the separately moving body parts or spare parts of the examined person, other living being or object; a control unit, data storage and the fixed or wireless connections between them. Another feature of the device is that, in case of modeling human motion, in addition to the measurement units placed on the thighs, lower legs, feet, shoulders, upper arms, forearms, hands, the head, the hip and the trunk, there is at least one more measurement unit on the back, fixed near the spine, and a sole unit is connected to the foot measurement units, and a pressure sensor glove is connected to the hand measurement units. The hip measurement unit and the spine measurement units are fixed to the user's back. In the sole unit, there are at least two pressure sensors, out of which one is placed at the corner of the foot and the other one is in the area under the toes. In the pressure sensor gloves there are at least three pressure sensors, located on the palm and on the inside of the fingertips.

The majority of measurement units is a standard measurement unit. In a general measurement unit, there is at least one three-axis acceleration sensor, at least one three-axis angular acceleration sensor, an internal data bus, a tuning three-axis magnetometer and an interface towards the control unit.

In the foot measurement units, there are 2-2 three-axis acceleration sensors, three-axis angular acceleration sensors, a tuning three-axis magnetometer, an interface towards the control unit, furthermore, analog-to-digital converters in a number corresponding to the number of the pressure sensors connected to the measurement units of the given foot measurement unit.

The analog-to-digital converters are connected to one of the pressure sensors.

In the hand measurement units, there are three-axis acceleration sensors, three-axis angular acceleration sensors, a tuning three-axis magnetometers, analog-to-digital converters in a number corresponding to the number of the pressure sensors of the pressure sensor glove connected to the measurement units of the given hand and an interface towards the control unit. The analog-to-digital converters are connected to one of the pressure sensors.

The control unit contains a main processor, a real-time clock circuit with an independent power source, a data storage and a USB connector and an interface towards the measurement units. The subunits of the control unit are connected by an internal data bus.

A preferred embodiment of the apparatus may be where the interface of the control unit and all the measurement units is an RF unit.

A preferred embodiment of the control unit is where it also has at least one external bus connector.

In a further preferred embodiment of the apparatus, the sole unit also has a third pressure sensor, located below the metatarsal bone.

Another advantageous embodiment of the equipment is characterized by the fact that at least a part of the measurement units is equipped with an independent internal processor, and the internal processors are connected to the acceleration and angular acceleration sensors of the given measurement unit, its magnetometers, analog-to-digital converters, directly or via an internal data bus.

In another preferred embodiment of the apparatus, the control unit forms a single mechanical unit with the hip measurement unit, and it is fixed around the hip, next to the spine, preferably by a lumbar belt or by integration into a garment worn on the body.

We also find it preferred if - in case the interface is a cable connection - the cables between the inertial units and the control unit are passed through an adjustable cable holder. The adjustable cable holder holds the cable tight at several points and the cable is guided through it so that, in the meantime, it forms two open loops in front of each other; by pulling the two free branches of the adjustable cable holder, the size of the loops can be reduced.

We also find it preferred if - in case the interface is a cable connection - the cables are wound up on a pre-tensioned drum.

The (general) measurement units between the shoulder blades and in the middle of the back are attached to a strap vest that consists of a horizontal round strap underneath the breast and a diagonal cross strap fitted to the back.

The two lower branches of the diagonal cross strap is attached to the round strap under the armpits, and its upper branches are connected to the breast part or under-the-armpit-part of the round strap going downwards above the breast, folded over the shoulder. The measurement unit between the shoulder blades is fixed at the intersection point of the upper and lower branches of the cross strap, and the central measurement units of the back are fixed to the rear side of the round strap.

We use illustrations for the further introduction of the patent:

1. Figure: the location of measurement units - spatial view;
2. Figure: the scheme of the motion digitizing and evaluating device,
3. Figure: the scheme of a general measurement unit,
4. Figure: the scheme of a general measurement unit with an internal processor,
5. Figure: the scheme of a unified control unit,
6. Figure: hand measurement unit scheme,
7. Figure: foot measurement unit scheme,
8. Figure: the layout of pressure sensors on the pressure sensor gloves, palm side view,
9. Figure: a pressure sensing glove with pressure sensor no. 14, palm side view,
10. Figure: top view of the sole unit,
11. Figure: another sole unit in top view,
12. Figure: spatial view of a cable holder with cable,
13. Figure: side view of the cable holder,
14. Figure: front view of the strap vest,
15. Figure: rear view of the strap vest.

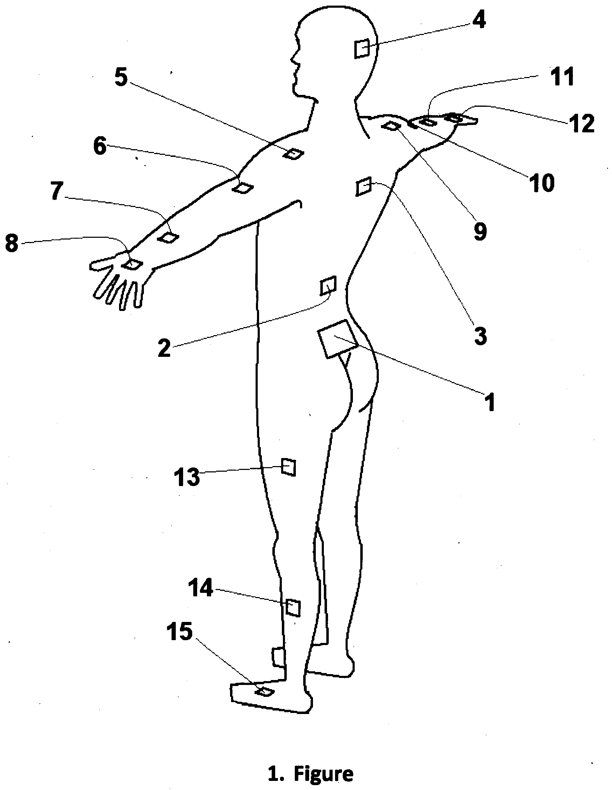

The device to be presented is presented through the description of the hardware and its operation. The basis of sensing is the sensor net incorporating at least 18+ measurement units, which is attached to the different parts of the human body by a special strap vest. Additionally, on the basis of the purpose of use of the digital motion recordings, further sensors can be integrated in any number. In Figure 1, the measurement unit placement order, recommended by us, is shown, according to the control unit (1) design according to Claim 7, wherein the control unit (1) is mechanically unified with a spine measurement unit, preferably with the hip measurement unit. The advantage of unification is the easier handling and the protection of the control unit (1), but, of course, the hip measurement unit could also be a general-design measurement unit in case of a separate control unit, or the control unit can also be integrated with another measurement unit. According to to our recommendation, in the evaluating software, the origin of the own coordinate system of the mapped model falls into this point, as well. The left foot measurement unit is fixed to the left foot, the left lower leg measurement unit (14) is fixed to the left lower leg, and so on, according to the names and the figure, they are followed by the head measurement unit (4), the left shoulder measurement unit (5), the left upper arm measurement unit (6), the left forearm measurement unit (7), the left head measurement unit (8), the right shoulder measurement unit (9), the right upper arm measurement unit (10), the right forearm measurement unit (11) and the right hand measurement unit (12), 13 left thigh measurement unit, 16 right thigh measurement unit, 17 right lower leg measurement unit, 18 right foot measurement unit, and 2 is the second spine measurement unit, furthermore, 3 is the third spine measurement unit. The majority of the measurement units is a general measurement unit, enabling a simpler and more uniform internal structure, but the structure of the hand measurement units no. 8 and 12, the foot measurement units no. 15 and 18 and, where appropriate, one of the spine measurement units are different from these, partly for a more accurate sensing and the related peripherals. In Figure 1 it can be seen that the movement of the strain is mapped with one more measurement unit that is not placed on the abdomen.

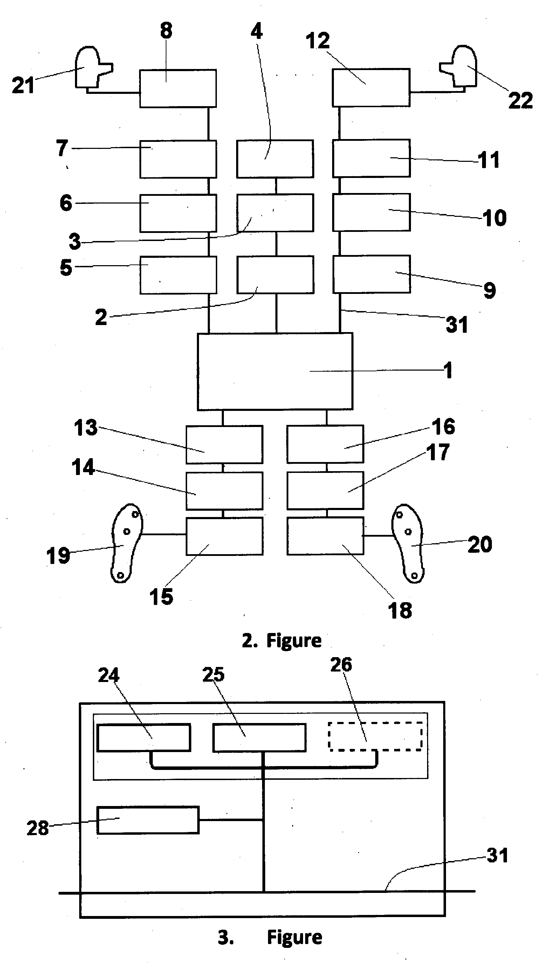

Figure 2 shows the measurement units together with their connections. In this figure, the new information is provided by the fact that the interface no. 31 has a cable-based design. By the interface no. 31 we mean the connections between the specific measurement units or the connection between them and control unit no. 1. It can also be seen in Figure 2 that the sole units no. 19 and 20 are connected to foot measurement units no. 15 and 18, and the pressure sensor gloves no. 21 and 22 are connected to hand measurement units no. 8 and 12. In Figure 3, the inner scheme of a simpler design of the general measurement units can be seen. These measurement units consist of a three-axis acceleration sensor (24), a three-axis angular acceleration sensor (25), a tuning, three-axis magnetometer (28) and within the measurement unit, they are interconnected with the interface (31), through which they can be connected to the control unit (1).

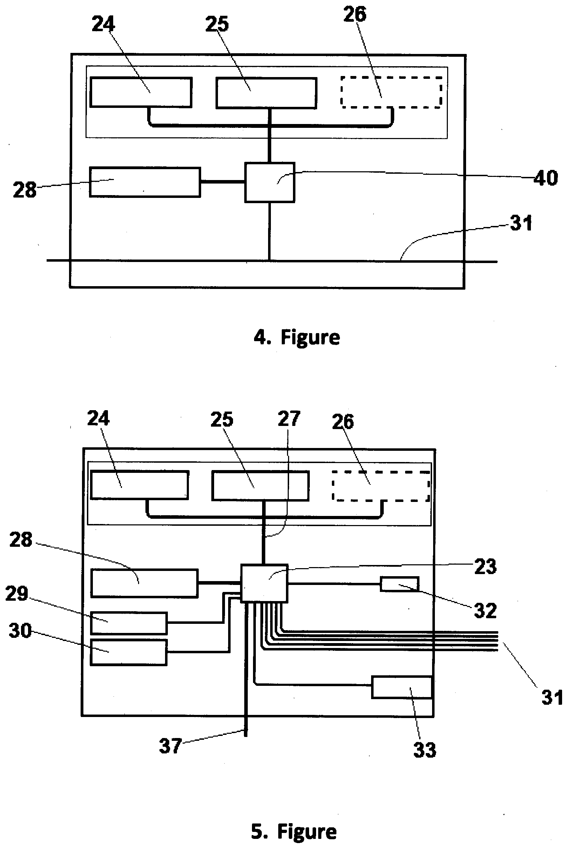

We illustrated a secondary, three-axis magnetometer (26) with an intermittent line, which is although not strictly necessary for the operation, but as this module is widely included in the sensor units manufactured for this purpose, we show it, as well. In industrial environments, this magnetometer sensing method is not reliable, therefore its signals are not even used. Figure 4 is the internal scheme of the general measurement units equipped also with an internal processor no. 40. A three-axis acceleration sensor (24), a three-axis angular acceleration sensor (25), a tuning, three-axis magnetometer (28) and a secondary, three-axis magnetometer (26) (which is not necessary here, either, according to the description of the previous Figure) is connected via an internal data bus (27) to the internal processor (40), which preliminarily processes the signals of the sensors, and then it transmits the data to the control unit (1) via the interface (31) connected to it. In Figure 5, again the internal structure of the control unit of unified design can be seen. According to the general measurement unit design, the measurement unit function is fulfilled by a three-axis acceleration sensor (24) and a three-axis angular acceleration sensor (25), a tuning, three-axis magnetometer (28) under the control of a larger-capacity main processor (23). Furthermore, a real-time clock circuit no. 32 is also connected to the main processor no. 23, which is supplemented by an independent power supply (e.g. super capacity), and its function is to ensure that the recordings can be accurately dated even when offline. The tuning three-axis magnetometer no. 28 assists the orientation and fine tuning at the beginning of the measurement, but it is no longer needed during the measurement. Control unit no. 1 is equipped with an RF unit no. 29, and also with a USB unit no. 33, with which the external data transmission or even the communication towards the measurement units can be realized. The most obvious implementation of the RF unit is a WiFi unit, but another radio frequency data transmission may also be suitable in a way obvious for a person skilled in this field.

If the RF unit no. 29 fulfils the communication with the measurement units, of course, there must be a suitable transceiver or transceiver-receiver module also in the measurement units. Data storage no. 30 serves to store the recording and allows the device to be used also in island mode. The data storage no. 30 is preferably an SD memory card. The connection to measurement units no. 2, 3, 4, 5, 6, 7, 8, 9, 10, 11, 12, 13, 14, 15, 16, 17 and 18 is provided by interface no. 31 (or a direct, cable connection in a simple case). An external data bus connector no. 37 can serve for other data transmission purposes, which is also connected to the main processor no. 23.

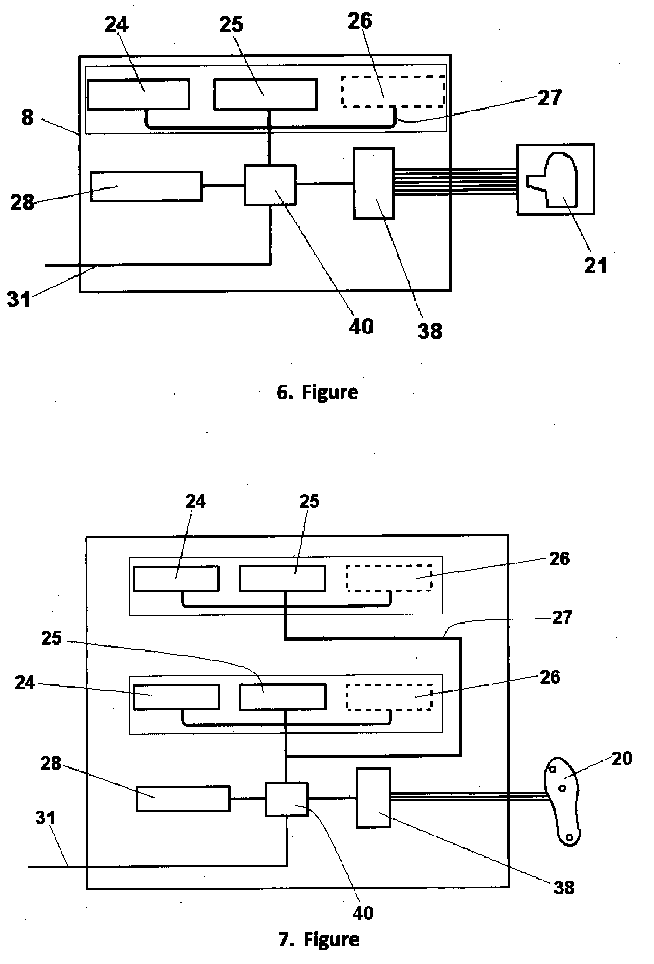

Figure 6 shows the structure of hand measurement units no. 8 and 12. Compared to the general measurement units, the main difference is that the pressure sensor gloves no. 21 and 22 are connected to the hand measurement units no. 8 and 12, containing at least three, but - for the sake of sufficient sensing - rather seven pressure sensors (44) per piece, the signals of which is received by analog-to-digital converters (38), which transmit them to the internal processor (40).

Figure 7 shows the structure of foot measurement units no. 15 and 18. Here, too, in the basic structure, we followed the pattern of the general measurement units, but for an even more accurate detection of the location-changing movements, a double inertial sensor set is used, preferably with 2-2 three-axis acceleration sensors (24) and a three-axis angular acceleration sensor (25) (and, a tuning, three-axis magnetometer (28), but there are the afore-mentioned external, material purchasing reasons for this, in general), connected to the internal processor (40) by the internal data bus (27). Sole units no. 19 and 20 are connected to the foot measurement units no. 15 and 18, and the signals of the pressure sensors no. 44 located in them are received by the analog-to-digital converter no. 38 of a number corresponding to their number, also in this case.

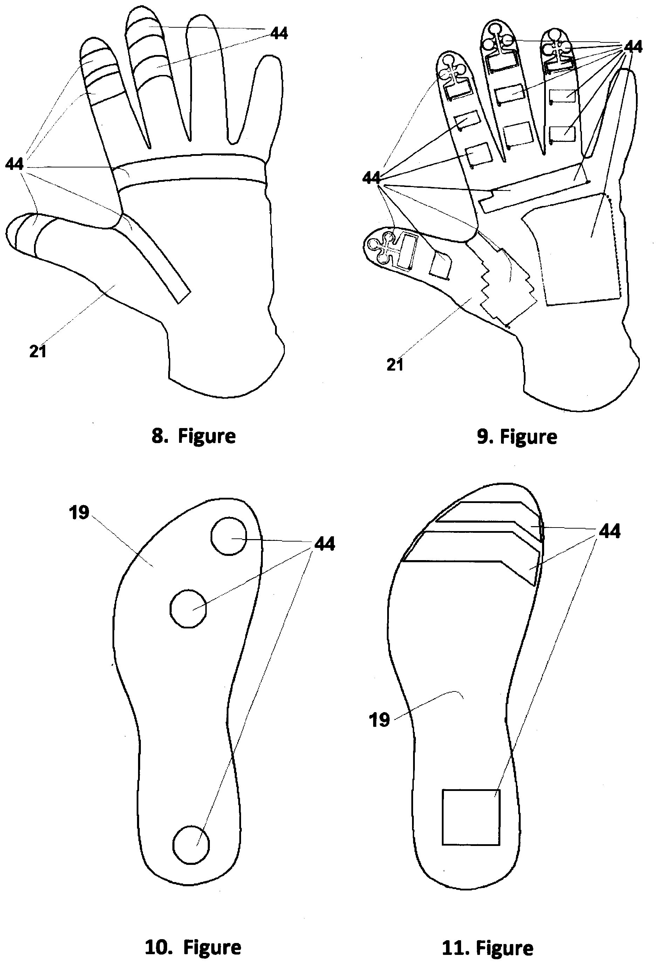

A useful element of the device is the left pressure sensor glove no. 21 that can be seen in Figure 8. We recommend to fix the pressure sensors no. 44 to the inner side, both on the thumb, the pointer and the center finger, and also on the palm. The *FSR408*-type sensor was found suitable for this. By using the pressure sensors no. 44, it is possible to estimate the force of rotation and measure the duration of the rotation with great precision.

The palm pressure measurement taking place on seven surfaces complies with all the criteria set by the ergonomic systems.

In Figure 9, we can see the inner side of a pressure sensor glove with a higher resolution, containing a total of fourteen pressure sensors no. 44. Here, with the exception of the little finger, almost all phalanxes have a pressure sensor (44), and also the palm is equipped with three, large-surface sensors (44), by which an ergonomic, lifelike holding sensation and high-precision sensing can be ensured at the same time. It is advisable to connect certain sensors, located e.g. on the same finger or on the palm not singularly, but in parallel with each analog-to-digital converter.

In Figure 10, the left sole unit no. 19 is shown in bottom view. Sole unit no. 19 is an insole that can be placed into a shoe or possibly onto the sole of the shoe. For the advanced step recognition recommended by us, on the sole unit (19), a pressure sensor (44) must be placed at least under the toes and under the heel, but it is even more preferable to place a third sensor under the metatarsal bone. In this place, the *FlexiForce A301*-type, independent, coinsized sensor is suitable, as well, and it does not cause any discomfort to the wearer if it is placed outside the shoe.

However, the AXS-TA1.0-type sole unit no. 19, which can be seen in Figure 11 can also be placed into shoes as an insole, as it is soft and the pressure sensors no. 44 are integrated into the fabric. The insole provides comfortable and ergonomic wear. Such an insole can be created in a film or sensor design with a conductive thread. In the case of the former, we stick a pressure-sensitive conductive sheet on a flexible circuit board, on top of which a foam (preferably a neoprene) layer is placed, and in case of the second version, we place a pressure-sensitive conductive sheet in between two external foam layers, so that a conductive thread (fabric) layer is placed in between the foam layers and the pressure-sensitive conductive sheet.

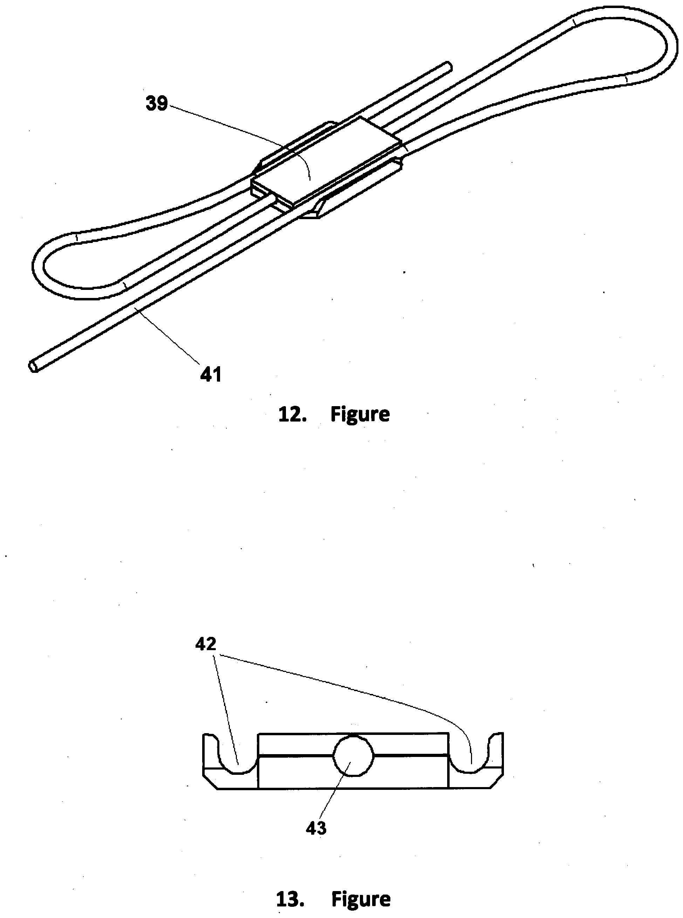

Figure 12 shows a cable holder no. 39 during operation. The connection between the measurement units can be cable-based and also wire-free, as mentioned above. If, in the previous case, we would like to ensure the use of the device in a wide range of sizes, in case of smaller-size models, there is a significant amount of freely moving cable amount on the body of the model, which is not beneficial from a work safety point of view. To solve the problem, we recommend a unique, dual, face-to-face open loop solution. At the two ends of the cable holder (39), 1-1 clamping slots (42) are placed, into which the cable part of the desired length (up to half a meter) can be fitted according to Figure 12, and the middle cable branch can be led through its middle borehole. Cable holder no. 39 consists of two halves. By pulling the free cable branches, the size of the open loops entangled by cable holder no. 39 is reduced, thereby one can increase the swinging cable length, while by pulling the loops, an opposite effect can be achieved.

In Figure 13, we can see the side view of cable holder no. 39. In the clamping slots no. 42, upon manual force, the cables trapped can move out of their position. Cable borehole no. 43 becomes accessible by disassembling the lower and upper half (snapping them off).

Other solutions, such as a cable drum pre-tensioned by a spring can also be used as a cable holder.

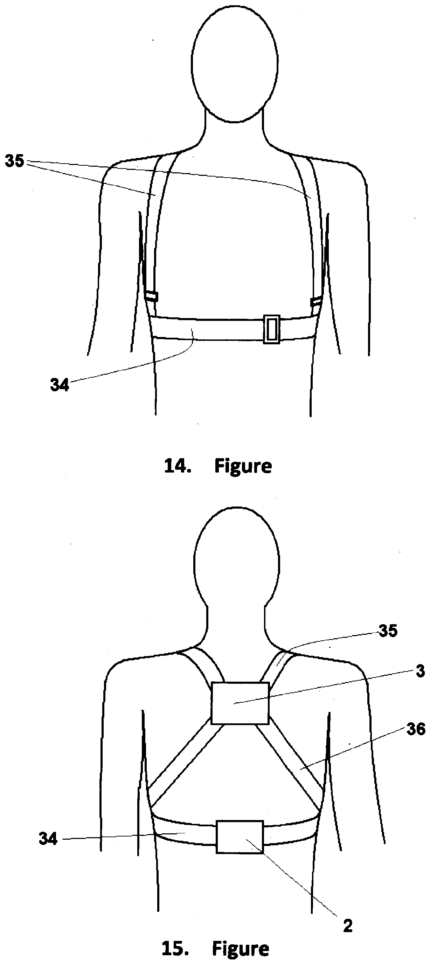

The task of the strap vest worn both in front view and rear view in Figure 14 and 15 is to hold the second spine measurement unit no. 2 and the third spine measurement unit no. 3 losely to the body (particularly along the spine). The strap vest consists of a round strap no. 34 surrounding the breast line, with an adjustable length, furthermore, the related diagonal cross-strap fitted to the back. The two lower branches of the diagonal cross strap are attached to the round strap (34) under the armpits, and its upper branches (35) are connected to the breast, outer part or the under-the-armpit-part of the round strap (34) going downwards above the breast, folded over the shoulder. The measurement units are fixed at the intersection point of the lower branches of the cross strap no. 36 and its upper branches no. 3, and to the rear part of round strap no. 34. Due to the increased number of measurement units no. 1, 2 and 3 linked to the spine to three and due to the stabilizing effect of the strap vest, also the rotation between the coccyx-Th12 and Th11-Th4 can be accurately detected, unlike in case of the solution that have been applied so far.

As a particularly important innovation, as a result of the special strap vest, all of measurement units no. 1, 2 and 3 lies directly on the spine and there is no one of them on the chest, so the evaluation data are much more accurate than those of the technologies where one out of the three measurement units are placed on the chest.

The processing software of the motion digitizing and evaluating device moves a simplified virtual bone system in the 3D space from the data aggregated and prepared by the control unit of the hardware, which is capable of displaying the recording even in real-time, and to ward about any deviation from the preset normal values by using colour codes depending on the extent o fthe deviation, or, in case of offline mode, to immediately play the motion after its recording and to evaluate it according to given criteria. The processing software preferably runs on a higher-capacity, separate hardware unit (e.g. PC, tablet, etc.), where data may be received via a cable connection or wireless connection or from a media removed from the control unit and inserted into the hardware unit.

Before digitally recording the motion, the sensor net placed on the subject must be calibrated. The mathematical model of the motion digitizing and evaluating software is based on the vertical posture (standing at attention), and then it tracks the changes of axes with the following formula: $Qi = Conjugate(Qbi) * Qci$. The main processor of the control unit gives a quaternion as a result from the data of the intertial measurement units. The applied mathematical model calculates with relative displacements (delta) relative to the initial state (basic position), which are derived from the difference between the angular displacements of the specific bones.

The forward direction is recorded in standing at attention (basic position). The center of the hip - determined in the basic position - is the origin of the coordinate system of the motion digitizing and evaluating system. The sum of the initial directions and lengths (vectors) constitutes the "model" in the local coordinate system. When using the special magnetometer of the control unit, the base orientation of the model can be orientated in relation to the northern direction.

The correction record is suitable for eliminating errors caused by dressing or for the subsequent correction of measurement errors. The limbs and the spine are vertical, and the shoulder and the sole are horizontal. On the basis of the gravitational vector, the deviation, the inclination compared to the horizontal and vertical level can be measured, so that the placement error can be corrected.

With the correction motion series, the calibrating person coordinates the sensors. As a result of the calibration process, the motion digitizing and evaluating software is functional and provides accurate results even without any magnetic orientation data.

Correction can be done in two ways. The first method, the gravitational methodology is based on the applied vector system. The body is described as a contiguous vector system where the endpoints of each vector is the starting point of the next vector, and the management of the endpoints is hereditary when implementing rotations. During the correction, certain elements of the vector system are rotated to a vertical position compared to the gravitational direction. In the second case, during the rotated methodology, by "reversing" the angular differences resulting from the placement and made highlighted by the supplementary or subsequent special correction movements, are corrected. The two correction procedures mutually exclude each other, both of them can be carried out, but only one of them can be applied at once, set per bone.

Basic position: QiB;

amended basic position: Qicb.

Mathematical interpretation:

The mathematical model based on the quaternion used differs from the methodologies described so far, and the processors used provide a quaternion afrer their internal data fusion. The pre-screening and fusion of data is performed by the chip itself.

$$Mi = Mprev * convertToMatrix(Conjugate(Qprev))$$

$$Qi = Conjugate(Qib) * Qic$$

optional compensation Qi= Qi * Qicb
optional rotation axis reversal Qi.X<->Qi.Z optional
rotation axis reversal Qi.Y<->Qi.Z optional rotation
axis reversal Qi.X<->Qi.Y optional rotation direction
change to axis X Qi.X = Qi.X * -1 optional rotation
direction change to axis Y Qi.Y = Qi.Y * -1 optional
rotation direction change to axis Z Qi.Z = Qi.Z * -1

$$Mi = Mi * convertToMatrix(Qi)$$

Qib = quaternion base (frame) for bone i. (standing at
attention) Qicb = quaternion Compensation base for
bone i. Qic = quaternion current (frame) for bone i.
Mi = transformation matrix 4x4 for bone i.
Mprev = the Mi calculated for the bone preceding it in the
kinematic chain Qprev = the Qi calculated for the bone preceding it in the kinematic chain

The calculation order of bones is in their kinematic order. The hip is the first bone, and all the rest are calculated relatively to it. In case of the first element of the kinematic chain, Mprev unit matrix, Qprev [1,0,0,0].

With the proposed motion digitizing and evaluating device, the motion of any live or movable body can be digitized. Irrespective of geographical location, it can be used anywhere, whether in water, under the ground level, above the ground level, in the air, because it is capable of autonomous data recording without any external equipment, and the number of simultaneously recordable subjects is not limited either. It can be used under magnetically disturbed (electromagnetic or ferromagnetic) circumstances, and in the autonomous mode, it is able to operate without emitting any radio frequency interference, so it does not interfere with the nearby devices using radio frequency.

*List of reference signs*

1- control unit
2- the second spine measurement unit
3- the third spine measurement unit
4- head measurement unit
5- left shoulder measurement unit
6- left upper arm measurement unit
7- left forearm measurement unit
8- left hand measurement unit
9- right shoulder measurement unit
10- right upper arm measurement unit
11- right forearm measurement unit
12- right hand measurement unit
13- left thigh measurement unit

14- left lower leg measurement unit

15- left foot measurement unit

16- right thigh measurement unit

17- right lower leg measurement unit

18- right foot measurement unit

19- left sole unit

20- right sole unit

21- left pressure sensor glove

22- right pressure sensor glove

23- main processor

24- three-axis acceleration sensor

25- three-axis angle acceleration sensor

26- secondary three-axis magnetometer

27- internal data bus

28- tuning three-axis magnetometer

29- RF unit

30- data storage

31- coupler surface

32- real-time clock circuit,

33- USB

34- round strap

35- upper branch

36- lower branch

37- external bus connector

38- analog-to-digital converter

39- cable holder

40- internal processor

41- cable

42- clamping slot

43- cable borehole

44- pressure sensor

## Claims

1. Device for digitizing and evaluating movement, consisting of the inertial measurement units (2, 3, 4, 5, 6, 7, 8, 9, 10, 11, 12, 13, 14, 15, 16, 17, 18) fixed to the separately moving body parts or spare parts of the examined person, living creature or object; a control unit (1), a data storage (30) and the fixed or wireless connections between them, **characterized by** the fact that in case of modeling human motion, in addition to the measurement units placed on the thighs, lower legs, feet, shoulders, upper arms, forearms, hands, the head, the hip and the trunk, there is at least one more measurement unit on the back, fixed near the spine, and a sole unit (19, 20) is connected to the foot measurement units (15, 18), and a pressure sensor glove (21, 22) is connected to the hand measurement units (8, 12); the hip measurement unit and the spine measurement units (2, 3) are fixed to the back of the user, in a sole unit (19, 20) there are at least two pressure sensors (44), one of the pressure sensors (44) is located at the heel, and the other pressure sensor (44) is located in the area under the toes; in the pressure sensor glove (21, 22) there are at least three pressure sensors (44), placed on the palm and the inside of the fingertips; with the exception of the foot measurement units (15, 18) and the hand measurement units (8, 12), the measurement units are general measurement units (2, 3, 4, 5, 6, 7, 9, 10, 11, 13, 14, 16, 17); in a general measurement unit (2, 3, 4, 5, 6, 7, 9, 10, 11, 13, 14, 16, 17), there is at least one three-axis acceleration sensor (24), at least one three-axis angular acceleration sensor (25), a tuning three-axis magnetometer (28) and an interface connecting the above (31) towards the control unit (1),
in the foot measurement units (15, 18) there are two pieces of three-axis acceleration sensors (24), two three-axis angular acceleration sensors (25), a tuning, three-axis magnetometer (28), an interface (31) towards the control unit (1) and the analog-to-digital converters (38) in a number corresponding to the number of the pressure sensors (44) connected to the measurement units (15 and 18) of the given foot are connected to a pressure sensor (44),
in the hand measurement units (8, 1.2) there are 1-1 three-axis acceleration sensors (24), two three-axis angular acceleration sensors (25), a tuning, three-axis magnetometer (28), analog-to-digital converters (38) in a number corresponding to the number of the pressure sensors of the pressure sensor glove unit connected to the given hand measurement unit (8, 12), and an interface (31) towards the control unit; the analog-to-digital converters (38) are connected to a pressure sensor (44),

the control unit (1) contains a main processor (23), a real-time clock circuit (32) with an independent power source, a data storage (30) and a USB connector (33) and a coupling surface (31) towards the measurement units (2, 3, 4, 5, 6, 7, 8, 9, 10, 11, 12, 13, 14, 15, 16, 17, 18), and the subunits of the control unit (1) are connected by an internal data bus (27).

2. A motion digitizing and evaluating device according to Claim no. 1, **charac terized by** the fact that if the interface (31) is a cable connection, the cables between the measurement units (2, 3, 4, 5, 6, 7, 8, 9, 10, 11, 12, 13, 14, 15, 16, 17, 18) and the control unit (1) are led through an adjustable cable holder (39); the adjustable cable holder (39) holds the cable tight at several points and the cable is guided through the cable holder (39) so that, in the meantime, it forms two open loops in front of each other; by pulling the two free branches of the adjustable cable holder (39), the size of the loops can be reduced.

3. A motion digitizing and evaluating device according to any of Claims no. 1-2, **characterized by** the fact that if the interface (31) is a cable connection, the cables are wound up onto a pre-tensioned drum.

4. A motion digitizing and evaluating device according to Claim no. 1, **charac terized by** the fact that the interface (31) of the control unit (1) and all the measurement units (2, 3, 4, 5, 6, 7, 8, 9, 10, 11, 12, 13, 14, 15, 16, 17, 18) is 1-1-RF unit (29).

5. A motion digitizing and evaluating device according to any of Claims no. 1-4, **characterized by** the fact that the control unit (1) also has at least one external bus connector (37).

6. A motion digitizing and evaluating device according to any of Claims no. 1-5, **characterized by** the fact that the sole units (19 and 20) also have a third pressure sensor (44) under the metatarsal bone.

7. A motion digitizing and evaluating device according to any of Claims no. 1-6, **characterized by** the fact that the control unit (1) forms a single mechanical unit with the hip measurement unit, the control unit (1) is fixed nearby the hip, next to the spine, preferably by a lumbar belt or by integration into a garment worn on the body.

8. A motion digitizing and evaluating device according to any of Claims no. 1-7, **characterized by** the fact that the second spine measurement unit (2) and the third spine measurement unit (3) are preferably fixed to a strap vest that consists of a horizontal round strap (34) under the breast and a diagonal cross-strap fixed to the back; the two lower branches (36) of the diagonal cross-strap are connected to the round strap (34) under the armpits, and its upper branches (35) are connected to the breast-side part of the round strap (34) over the shoulder, above the breast in downward direction, or to the under-the-armpit part; the second spine measurement unit (2) is fixed at the intersection point of the lower branches (36) and the upper branches (35) of the cross-strap, and the spine measurement unit (3) is fixed to the rear part of the round strap (34).

9. A motion digitizing and evaluating device according to any of Claims no. 1-8, **characterized by** the fact that at least a part of the measurement units (2, 3, 4, 5, 6, 7, 8, 9, 10, 11, 12, 13, 14, 15, 16, 17, 18) is equipped with an independent internal processor (40), and the internal processors (40) are connected to the acceleration and angular acceleration sensors (24, 25) of the given measurement unit, and to the magnetometers (26, 28) and analog-to-digital converters (38) of the given measurement unit, directly or through an internal data bus (27).

**1. Figure**

2. Figure

3.    Figure

EP 3 401 873 A1

4. Figure

5. Figure

12

**24** **25** **26**

**27**

**8**

**28**

**31**

**40** **38**

**21**

6. Figure

**24** **25**

**26**

**27**

**25**

**24**

**26**

**28**

**20**

**31**

**38**

**40**

7. Figure

44

44

21

**8. Figure**

44

44

21

**9. Figure**

19

44

**10. Figure**

44

19

**11. Figure**

39

41

12. Figure

42

43

13. Figure

EP 3 401 873 A1

**14. Figure**

**15. Figure**

16

## INTERNATIONAL SEARCH REPORT

| International application No. |
|---|
| PCT/HU 2015/000078 |

| A. CLASSIFICATION OF SUBJECT MATTER |
|---|
| G06Q 50/22 (2012.01); A61B 5/103 (2006.01) |
| According to International Patent Classification (IPC) or to both national classification and IPC |

| B. FIELDS SEARCHED |
|---|
| Minimum documentation searched (classification system followed by classification symbols) |
| G06Q 50/00, 50/22, G09B 19/00, 19/10, G01P 21/00, G01R 33/00, G09G 5/00, G06F 19/00, A61B 5/00, 5/103, A63F 13/00 13/20 13/21 13/211 13/212 3/218 |
| Documentation searched other than minimum documentation to the extent that such documents are included in the fields searched |
| |
| Electronic data base consulted during the international search (name of data base and, where practicable, search terms used) |
| PatSearch (RUPTO internal), USPTO, PAJ, K-PION, Esp@cenet, Information Retrieval System of FIPS |

| C. DOCUMENTS CONSIDERED TO BE RELEVANT |
|---|

| Category* | Citation of document, with indication, where appropriate, of the relevant passages | Relevant to claim No. |
|---|---|---|
| Y | RU 2107328 C1 (LATYPOV NURAKHMED NURISLAMOVICH et al.) 20.03.1998, items 20-21, 23, 28, p. 7, lines 2-45, p. 8, lines 15-45, p. 9, lines 20-42, p. 11, line 52- p. 12, line 6 | 1-4 |
| Y | US 2015/0149104 A1 (JOHN BAKER et al) 28.05.2015, para. [0045], [0084], [0085], [0113], item 1 of the claims | 1-4 |
| Y | US 6275213 B I (VIRTUAL TECHNOLOGIES, INC.) 14.08.2001, column 4, lines 16-27, column 9, lines 23-65, column 13, lines 40-64 | 1-4 |
| Y | US 8467979 V2 (ALLUVIAL JOULES, INC.) 18.06.2013, column 2, line 64- column 3, line 54 | 1-4 |
| Y | SU 1768136 A1 (SHCHNTRALNGI NAUCHNO-ISSLEDOVAETLSKY INSTITUT TRAVMATOLOGII I ORTOPEDII IM. N.N. PRIOROVA) 15.10.1992, column 3, lines 53-61 | 1-4 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 June 2016 (08.06.2016) | 23 June 2016 (23.06.2016) |
| Name and mailing address of the ISA/ RU | Authorized officer |
| Facsimile No. | Telephone No. |

Form PCT/ISA/210 (second sheet) (April 2005)

## INTERNATIONAL SEARCH REPORT

International application No.

PCT/HU 2015/000078

C (Continuation).    DOCUMENTS CONSIDERED TO BE RELEVANT

| Category* | Citation of document, with indication, where appropriate, of the relevant passages | Relevant to claim No. |
|---|---|---|
| Y | «TeddySling». Ergonomichnye riukzaki [on-line], April 2014 [retrieved on 20.04.2016]. Found on the Internet: <URL:http://web. archive.org/web/20140401100010/http://www.ya-na-mame.ru/ergon omichnie-ryukzachki/teddisling-teddysling/ergonomichniy-ryukzak-t eddisling-mechta/, p.1-4 | 2 |
| Y | «Cavo Retraibile Dati Caricabatterie per Iphone 4S 4 Ipod Touch 3G 4G Nano Ipad» [on-line], June 2015 [retrieved on 20.04.2016]. Found on the Internet: <http://web.archive.Org/web/20150604224 220/http://www.amazon.it/Cavo-Retraibile-Caricabatterie-Iphone-To uch/dp/B004WWWSKC>, p.1 | 3 |

Form PCT/ISA/210 (continuation of second sheet) (April 2005)

## INTERNATIONAL SEARCH REPORT

| International application No. |
| --- |
| PCT/HU 2015/000078 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☒ Claims Nos.: 5-8
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.  Consequently, this international search report is restricted to the invention first mentioned in the claims; it is covered by claims Nos.:

**Remark on Protest**       ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

**EP 3 401 873 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2787456 A2
- US 2007250286 A1
- US 2013217352 A1
- WO 2012161407 A1